(19) Europäisches Patentamt
European Patent Office
Office européen des brevets

(11) Publication number: **0 455 280 A1**

## (12) EUROPEAN PATENT APPLICATION

(21) Application number: 91200721.8

(22) Date of filing: 27.03.91

(51) Int. Cl.5: **C12N 15/31**, C12N 15/62, C12N 15/52, C12N 1/21, //(C12N1/21,C12R1:46)

The title of the invention has been amended (Guidelines for Examination in the EPO, A-III, 7.3).

(30) Priority: 30.03.90 NL 9000753

(43) Date of publication of application:
06.11.91 Bulletin 91/45

(84) Designated Contracting States:
BE DE DK FR GB NL

(71) Applicant: NEDERLANDS INSTITUUT VOOR ZUIVELONDERZOEK
Kernhemseweg 2
NL-6718 ZB Ede(NL)

(72) Inventor: van Asseldonk, Martinus Gerardus Josephus
Lijsterbeslaan 12
NL-3927 AE Renswoude(NL)
Inventor: de Vos, Willem Meindert
Langhoven 50
NL-6721 SJ Bennekom(NL)
Inventor: Simons, Augustinus Franciscus Maria
Durendaal 17
NL-6715 JP Ede (Gld)(NL)

(74) Representative: de Bruijn, Leendert C. et al
Nederlandsch Octrooibureau
Scheveningseweg 82 P.O. Box 29720
NL-2502 LS 's-Gravenhage(NL)

(54) DNA fragment from lactococcus lactis and fusion proteins thereof.

(57) The invention relates to a DNA fragment comprising at least part of the sequence which is located upstream of the sequence coding for the "mature" fragment of the extracellular protein having an apparent molecular weight of 60 kDa from Lactococcus lactis strains, in particular from the Lactococcus lactis ssp Lactis strain MG 1363.

Above DNA fragment may comprise one or two signal sequences coding for very effective signal peptides as well as a promotor sequence and a ribosome binding site sequence. These components of the DNA fragment in question may be either separately or jointly combined with a gene of a homologous or heterologous protein which then can be brought to expression via a host obtaining the desired extracellular homologous or heterologous protein.

EP 0 455 280 A1

# fig - 4  (1)

```
                                                                    KpnI
                                                      GGTACCAGTGAGTCATTTATTTTTGATTTTTCTAATGTATAGTG
TCACTCAGCACAGGCCAAGCTATATCATTATCTTAGCGGCGATTTTGGGTGCAATCTATGATAGCTATTATTTAGGTATTTACGGAATTGCGACCTTATTGTTCCCACTTATTGCTCTTT
TTGTATATAATATACAAATAACTATATTTACTAATCGCTGGACAAGGCTTTTTACAACAATTATTATTGTGACCGCTTTTGAAGTTTTTAGTGCAATCATTATGACAGCTTTTGGATTTG
CCCAACTTCAGTTTATCAAATTTGTTGTTTACCAGTTAGCGCCTACACTTTTGCTCAATATTATCTTAGCTGTAGCCTTACAATTCCCTTTAGAAATCTTTTACAGATTAAAGAAAAGTC
                   HaeII                             SstI
                                         SspI
ATGTAAGATACAATTAGAAAGTGTTTGTAATCATAAAGAAATATTAAGGTGGGGTAGGAAATAGTATAATATGTTTATTCAACCGAACTTAATGGGAGGAAAAATTAAAAAAGAACAGTT
1                                                                  ⋏
ATGAAAAAAAAGATTATCTCAGCTATTTTAATGTCTACAGTGATACTTTCTGCTGCAGCCCCGTTGTCAGGTGTTTACGCTGACACAAACTCAGATATTGCTAAACAAGATGCGACAATT
 1   M K K K I I S A I L M S T V I L S A A A P L S G V Y A D T M S D I A K Q D A T I
121
TCAAGCGCGCAATCTGCTAAAGCACAAGCACAAGCACAAGTTGATAGCTTGCAATCAAAAGTTGACAGCTTACAACAAAAGCAAACAAGTACTAAAGCACAAATCGCTAAAATCGAAAGC
41   S S A Q S A K A Q A Q A Q V D S L Q S K V D S L Q Q K Q T S T K A Q I A K I E S
241
GAAGCTAAAGCACTTAATGCTCAAATTGCTACTTTGAACGAAAGTATCAAAGAACGTACAAAGACATTGGAAGCTCAAGCACGTAGTGCTCAAGTTAACAGCTCAGCAACAAATTATATG
61   E A K A L N A Q I A T L N E S I K E R T K T L E A Q A R S A Q V N S S A T N Y M
361
GATGCTGTTGTTAATTCAAAATCTTTGACAGATGTTATTCAAAAAGTAACAGCTATTGCTACTGTTTCTAGTGCCAACAAACAAATGTTGGAACAACAAGAAAAAGAGCAAAAAGAGCTT
121  D A V V N S K S L T D V I Q K V T A I A T V S S A N K Q I L E Q Q E K E Q K E L
481
AGCCAAAAGTCAGAAACTGTTAAAAAGAACTACAACCAGTTCGTTTCTCTTTCACAAAGTTTGGATTCTCAAGCTCAAGAATTGACTTCACAACAAGCTGAACTCAAAGTTGCGACTTTG
161  S Q K S E T V K K N Y N Q F V S L S Q S L D S Q A Q E L T S Q Q A E L K V A T L
```

# fig - 4  (2)

```
601
AACTATCAAGCAACAATTGCAACTGCGCAAGATAAAAAACAAGCTTTATTAGATGAAAAAGCAGCTGCAGAAAAAGCAGCTCAAGAAGCAGCTAAAAAACAAGCGGCTTATGAAGCTCAA
301  N Y Q A T I A T A Q D K K Q A L L D E K A A A E K A A Q E A A K K Q A A Y E A Q
721
CAAAAAGAAGCAGCACAAGCACAAGCAGCTTCAACAGCAGCAACTGCTAAAGCTGTAGAAGCAGCAACTTCATCAGCTTCTGCTTCATCTAGTCAAGCTCCACAAGTAAGTACAAGCACT
241  Q K E A A Q A Q A A S T A A T A K A V E A A T S S A S A S S S Q A P Q V S T S T
841
GATAATACAACATCAAATGCTAGTGCCTCAAACAGTTCTAATAGTTCATCAAAACTCAAGTTCAAGTTCTAGCAGTTCATCAAGCTCAAGCTCAAGCTCAAGTAATTCTAATGCTGGTGGG
281  D N T S N A S A S N S S N S S S N S S S S S S S S S S S S S S S N S N A G G
961
AATACAAATTCAGGCACTAGTACTGGAAATACTGGAGGAACAACTACTGGTGGTAGCGGTATAAATAGTTCACCAATTGGAAATCCTTATGCTGGTGGTGGATGTACTGACTATGTATGG
321  N T N S G T S T G N T G G T T T G G S G I N S S P I G N P Y A G G G C T D Y V W
1081
CAATACTTTGCTGCACAAGGAATTTATATCAGAAATATCATGCCTGGTAATGGTGGACAATGGGCTTCTAATGGACCTGCCCAAGGCGTGCTCCATGTTGTAGGAGCTGCTCCTGGTGTT
361  Q Y F A A Q G I Y I R N I M P G N G G Q W A S N G P A Q G V L H V V G A A P G V
1201
ATCGCATCAAGCTTCTCAGCTGATTTTGTTGGATATGCAAACTCACCTTACGGTCACGTAGCTATTGTAAAATCAGTTAATTCAGATGGTACAATTACTATCAAAGAAGGCGGATATGGT
401  I A S S F S A D F V G Y A N S P Y G H V A I V K S V N S D G T I T I K E G G Y G
1321
ACAACTTGGTGGGACATGAACGTACTGTAAGTGCCGTCTGGTGTTTACTTTCTTGATGCCAAAACTAGAAAAAAGTCTTAATAAATAAAAAATAGTGGTTTGATAGTGGGGAATAATTTTCC
441  T T W W G N E R T V S A S G V T F L N P N           ----------------->       <-
1441
TTCTGTCAAATCATTTTTTATTATTGTGGTATAATAATAAGGAAAAATGATA
----------------
```

EP 0 455 280 A1

The invention relates, inter alia, to DNA fragments which at least code for part of the extra-cellular protein having an apparent molecular weight of 60 kDa from Lactococcus lactis strains such as the Lactococcus lactis ssp. lactis strain MG1363.

As is known, lactococci (Lactococcus lactis ssp. lactis and L.lactis ssp. cremoris) are used on a large scale as starter cultures in the preparation of dairy products such as cheese, butter and buttermilk. Recently, the complete nucleotide sequence of the genes for two extracellular proteins, i.e. proteinase prtP - [Vos et al., J. Biol. Chem. 264 (1989b), 13579-13585; Kok et al., Appl. Environ. Microbiol. 50 (1985), 94-101 and De Vos et al., Gene 85 (1989), 169-176] and the bacteriocin nisin [Buchman et al., J. Biol. Chem. 263 - (1988), 16260-16266] has been determined. The proteinase is synthesised as a pre-protein with a consensus ala/ala signal peptidase I cleavage site between the amino acid residues 33 and 34. It has been demonstrated that the signal peptide controls the secretion of heterologous proteins in lactic acid bacteria [Simons et al., Proceedings 2nd Netherlands Biotechnology Congress, (1988), 183-187]. The proteinase displays a significant sequence homology with a number of serine proteinases of the subtilisin family and also contains a C-terminal membrane anchor [Vos et al., 1989b loc.cit].

The gene for the bacteriocin nisin codes for a precursor polypeptide comprising 57 amino acids and having a molecular weight of 7500. A signal peptide comprising 23 amino acids is derived therefrom, which deviates from the consensus signal peptide rules as drawn up by Von Heijne, J. Mol. Biol. 184 (1985), 99-105. A series of post-translational modifications, including dehydration of serine and threonine residues and cross-linking with cysteine residues are required in order to obtain biologically active nisin.

Partly in view of the above, the Applicant has attempted to gain more insight into the characteristics and the structure of extracellular proteins of lactococci. This study was carried out, in particular, to establish optimum conditions for obtaining extracellular proteins, in particular homologous and heterologous extracellular proteins with lactococci as host.

The Applicant's study showed, as an unexpected result, that the Lactococcus strains involved in the study all secrete a protein with an apparent molecular weight of about 60 kDa, which was produced in relatively large amounts. This "60 kDa protein", which is the precursor of a 45 kDa "mature" segment of 434 amino acids with an amino acid composition and distribution which deviates considerably from that of generally known proteins, is designated "Usp45" in the framework of the invention. The gene of this protein of the Lactococcus lactis ssp. lactis strain MG1363 has been investigated and elucidated by the Applicant. More particularly, the nucleotide sequence of the "usp45" gene was found to possess, inter alia, an open reading frame of 1384 base pairs, which codes for a protein having 461 amino acids. The "mature" fragment, which starts with Asp at position 28 [D(28)], is preceded by a signal sequence coding for a signal peptide having 27 amino acids. Upstream the gene also contains a consensus promoter sequence and a ribosome binding site sequence; these components of the usp45 gene can be either separately or jointly combined with a gene of a homologous or heterologous protein, which can then be brought to expression via a host, obtaining an extracellular homologous or heterologous protein.

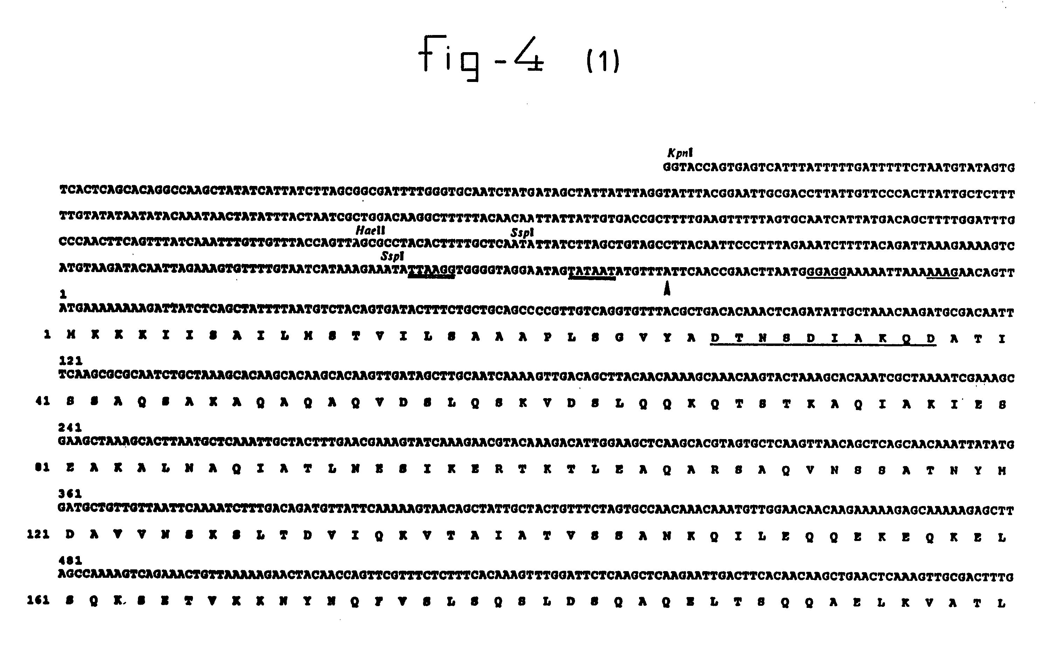

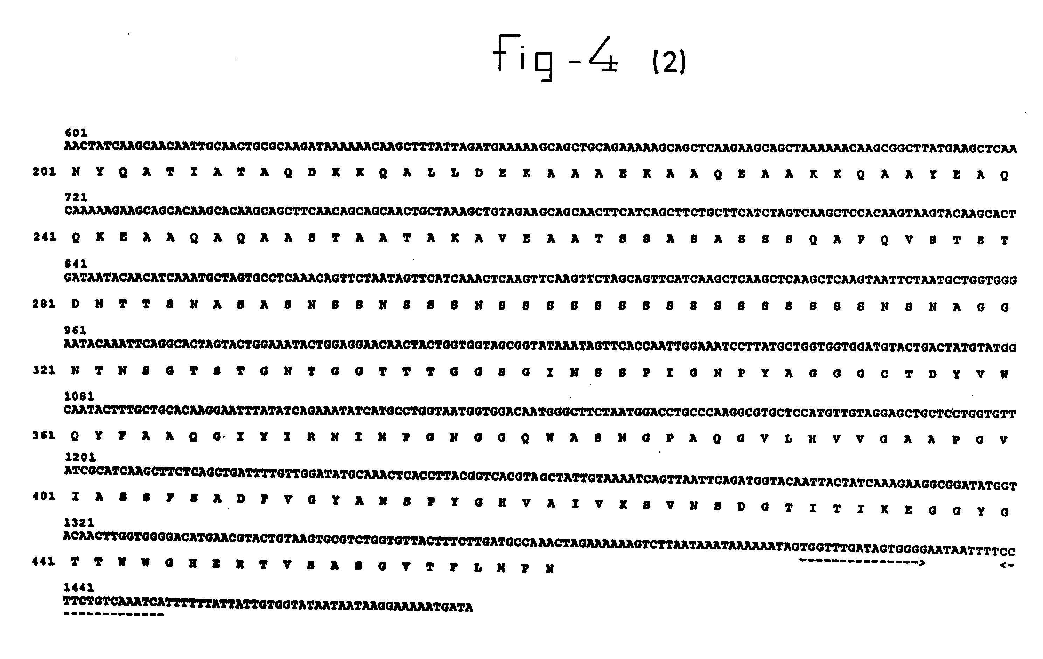

The invention therefore firstly relates to a DNA fragment which comprises at least a part of the sequence which is located upstream of the sequence coding for the "mature" fragment of the extracellular protein having an apparent molecular weight of 60 kDa of Lactococcus lactis strains such as the Lactococcus lactis ssp. lactis strain MG1363. More particularly, the DNA fragment comprises at least a part of the sequence located upstream of the sequence coding for the "mature" fragment of the extracellular 60 kDa protein, which "mature" fragment starts at D(28) in accordance with Fig. 4.

Advantageously, the DNA fragment in question comprises at least the signal sequence which codes for M (1) to A (27) inclusive, as shown in Fig. 4. In addition, the DNA fragment indicated above can also comprise the sequence coding for the "mature" fragment of the extracellular 60 kDa protein of Lactococcus lactis strains, such as, for example, the total sequence as shown in Fig. 4, which shown sequence originates from the Lactococcus lactis ssp. lactis strain MG1363.

Since the essence of the present invention essentially lies in the preparation of homologous and heterologous proteins in hosts acceptable for foodstuffs, such as lactococci, the invention in particular relates to DNA fragments which comprise at least a part of the sequence which - as indicated above - is located upstream of the sequence coding for the "mature" fragment of the extracellular 60 kDa protein of Lactococcus lactis strains, such as the Lactococcus lactis ssp. lactis strain MG1363, and also an extra sequence which codes for a homologous or heterologous protein. Examples of suitable homologous proteins, which frequently are of interest from the commercial standpoint, are, inter alia, components of the proteolytic system of Lactococcus lactis such as proteinases and peptidases. Examples which may be mentioned of suitable heterologous proteins are, inter alia, chymosin, prochymosin and pseudo-chymosin as well as α-amylase.

Since the abovementioned DNA fragments with the desired codings have to be brought to expression, the invention also relates to plasmids, which contain at least one abovementioned DNA fragment in hosts which contain a suitable plasmid according to the invention or an abovementioned DNA fragment in the genome, and also to the proteins obtained by culturing the modified hosts.

The homologous and heterologous proteins prepared via the hosts acceptable for the foodstuffs industry, such as lactic acid bacteria and the like, can be used in the manner customary for the use of these proteins. For example, the heterologous protein prochymosin can be converted by means of a method known from the prior art to chymosin, which can carry out a treatment required in cheese preparation.

The invention is illustrated in more detail with reference to the test results below.

## MATERIALS AND METHODS

### (a) Bacteria strains, phages and plasmids

The strains indicated in Table A below are used in the study according to the invention.

### TABLE A

Bacteria strains

| Strain | Characteristics | Source or reference |
|---|---|---|
| E.coli | | |
| JM83 | ara rpsL Δ (lac-proAB) | Messing (1983)[*] |
| JM103 | supE thi Δ (lac-ProAB) | Messing (1983)[*] |
| MB406 | supE recB22 recC21 sbcB15 | W.Dove, University of Wisconsin, USA |
| L.lactis ssp lactis | | |
| MG1363 | Plasmid-free | Gasson (1983)[**] |
| IL1403 | Plasmid-free | Chopin et al. (1984) |
| RI | | NIZO[***] |
| NZ22186 | Nis$^+$ Suc$^+$ | NIZO[***] |
| NCD0176 | Diacetylactis var. | National Collection of Dairy Organisms (NCDO) |
| ssp cremoris | | |
| SK110 | Prt$^+$ Lac$^+$ | De Vos & Davies, (1984)[****] |
| NCD01200 | Prt$^+$ Lac$^+$ | NCDO |
| WG2 | Prt$^+$ Lac$^+$ | NIZO[***] |

[*]    Methods in Enzymology 101 (1983), pp. 20-78.

[**]    J. Bacteriol. 154 (1983), pp. 1-9; also filed with the Centraal Bureau voor Schimmelcultures (Central Office for Mould Cultures, Baarn, The Netherlands, under No. CBS 364.89

[***]    Applicant

[****]    In 3rd European Congress on Biotechnology, Vol. III, Verlag Chemie, Weinheim 1984, pp. 201-205.

The lambda-phage 47.1 was used as cloning vector for the construction of a genome library [Loenen & Brammar, Gene 10 (1980), 249-259]. The further sub-cloning was carried out in pUC19 [Vierra and Messing, Gene 19 (1982), 259-268] and in M13mp18/mp19 [Norrander et al., Gene 26 (1983), 101-106].

### (b) Media, enzymes and chemicals

The lactococci were cultured on a medium based on whey: 5% whey permeate, 0.5% casiton (Difco), 1.9% $\beta$-glycerophosphate and 0.5% glucose. For the culturing of Lactobacillus-, Leuconostoc- and Streptococcus-strains, the casiton was replaced by 1.5% yeast extract (Difco).

The media which were used for E.coli were "Luria broth" (L-broth) [Miller, Cold Spring Harbor Laboratory, Cold Spring Harbor, NY, 1972] and trypticase peptone [Baltimore Biological Laboratories] in the form of a suspension or in solid form with 1.5% agar. Supplements were added in the following concentrations:

Carbenicillin [Beecham Pharmaceuticals] 100 μg/ml, X-Gal [5-bromo-4-chloro-3-indolyl-β-D-galac-topyranoside;

Boehringer] 40 μg/ml and IPTG [isopropyl-β-D-thiogalactopyranoside; Bethesda Research Laboratories, (BRL)] 25 μg/ml.

Restriction endonucleases and "reverse transcriptase" were obtained from BRL. T4 DNA ligase, polynucleotide kinase were obtained from New England Biolabs and Sequenase from United States Biochemical Corporation. [α³²P]dATP and [γ ³²P]dATP (3000 Ci/mmol) were obtained from Amersham. The oligonucleotides were synthesised using a Biosearch Cyclone DNA synthesiser [New Brunswick Scientific Corp.].

### (c) Construction and screening of a genome library of L.lactis ssp. lactis MG1363

The chromosomal DNA was extracted as follows:

L.lactis cells were cultured until $A_{600}$ = 1.0 and protoplasted in THMS buffer (25% sucrose, 30 mM Tris-HCl, pH = 8.0, 3 mM MgCl₂) with 2 mg/ml lysozyme [Sigma] for 1 hour at 37°C. After centrifuging (6000g), the protoplasts were resuspended in 1/20 volume TE (10 mM Tris-HCl, pH = 8.0, 1 mM EDTA) and extracted 3-4 times with phenol. The DNA was then dialysed against TE.

A genome library was constructed in the manner described by Maniatis et al., [Molecular cloning, a laboratory manual, Cold Spring Harbor Laboratory, Cold Spring Harbor, N.Y. 1982] in phage lambda 47.1 using "packaging" mixtures from Promega. The library was incorporated in E.coli strain MB406 and replicas were produced on nitrocellulose filters [Schleicher and Schuell]. The filters were blocked for 1 hour in PBS buffer (10 mM sodium phosphate, pH = 7.0, 0.85% NaCl) containing 1% bovine serum albumin (BSA) and incubated for at least 4 hours with antibodies against the 60 kDa protein in the PBS buffer containing 0.1% BSA. The blots were then incubated with Swine anti rabbit peroxidase conjugate (Swarpo) (Dakopatts). The proteins were rendered visible by incubating in substrate solution (40μg of 4-chloro-1-naphthol in 100 ml of 50 mM Tris-HCl, pH = 7.5, containing 30μl of H₂O₂).

### (e) RNA extraction

L.lactis ssp. lactis strain MG1363 cells were cultured at 30°C up until $A_{600}$ = 0.4-0.6. The cells were protoplasted in THMS buffer with 10 mg/ml of lysozyme for 10 min on ice and centrifuged. The RNA was extracted from the protoplasts with the aid of the hot-phenol method [Markham et al., J. Mol. Biol. 178 - (1984), 237-248].

### (f) Protein purification

Extracellular proteins of L.lactis ssp. lactis strain MG1363 were analysed using SDS/PAGE (sodium dodecyl sulphate/polyacrylamide gel electrophoresis) [Laemmli, Nature 227 (1970), 680-685]. The 60 kDa protein was excised from the gel and recovered by isotachophoresis [Öfverstedt et al., Anal. Biochem. 134 - (1983), 361-365].

### (g) Construction of a secretion vector for the production of prochymosin

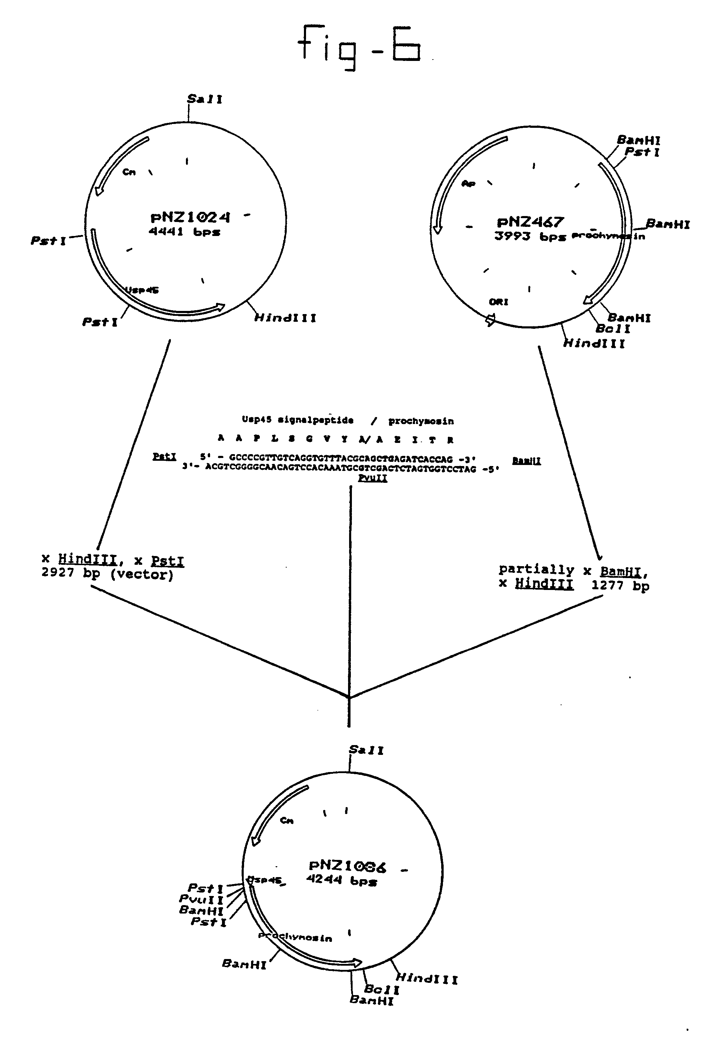

The experimental steps for constructing a secretion vector based on the regulatory sequences of the usp45 gene and the prochymosin gene is outlined in Fig. 6. Starting vector was pNZ1024, a derivative of pNZ123 wherein the SspI-ClaI fragment (nt -77 until nt 1544) of the usp45 gene was cloned. Synthetic linkers were constructed, from the PstI site at nucleotide position 56 of the usp45 gene to the first BamHI site at the amino terminal end of the bovine prochymosin gene, encoding a fusion between the coding region of the signal peptide of the usp45 gene and prochymosin. The synthetic linkers were designed in order to create a PvuII site at the end of the coding region for aa 1-27. Plasmid pNZ1024 was cut with PstI and HindIII. A vector (pNZ467) containing the prochymosin gene was partially digested with BamHI and subsequently cut with HindIII. The prochymosin gene in combination with the synthetic linkers were ligated into the cut vector to generate pNZ1086.

### (h) Construction of a secretion vector for the production of α-amylase

The construction of the α-amylase secretion vector is outlined in Fig. 7. Plasmid pNZα14 contains the α-amylase gene of Bacillus stearothermophilus ATCC12980 in which the T residue at position 102 was changed into a G residue creating an EagI site. This EagI site is located at the junction between the coding region of the signal peptide and the mature part of the α-amylase gene. After EagI digestion of this vector and filling-in the recessed ends with the Klenow fragment of DNA polymerase I in the presence of dGTP followed by a mung bean nuclease digestion and HindIII digestion, the coding region for the mature α-amylase could be ligated to pNZ1086 which had been digested with PvuII and HindIII, generating pNZ10α1. This vector codes for a perfect Usp45 signal peptide - mature α-amylase fusion.

In addition, a second secretion vector for the production of α-amylase was constructed by extending the DNA region upstream of the -35 region of the usp45 promoter sequence until the HaeII site at nucleotide position -198. This vector was designated pNZ10αA and contains an amino terminal extension of 122 nucleotides as compared to pNZ10α1.

## RESULTS AND DISCUSSION

### (a) Characterisation of secreted proteins from lactococci

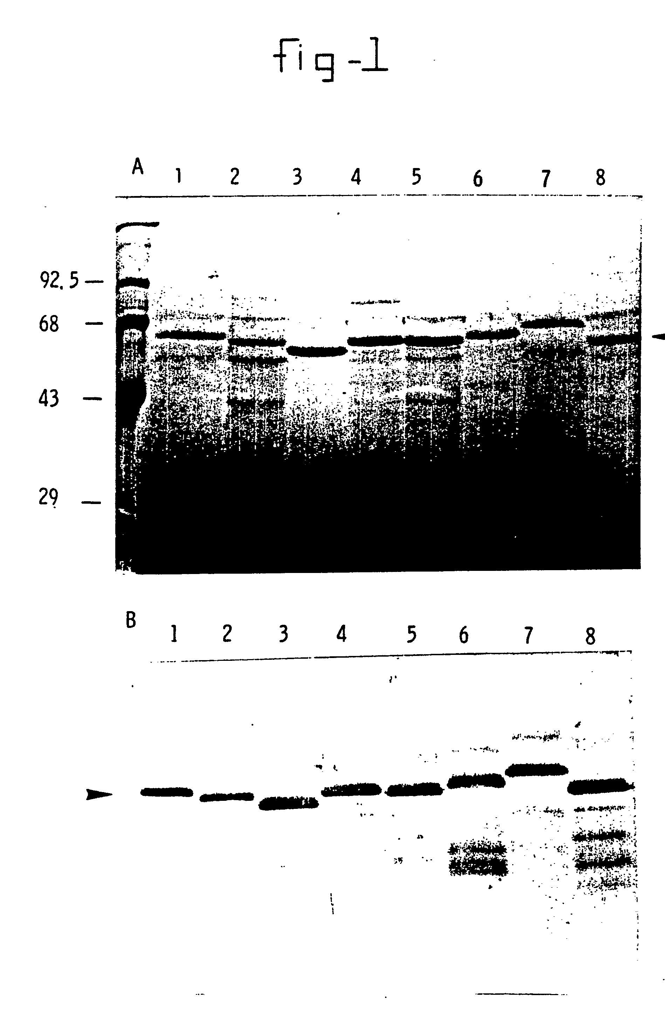

L.lactis ssp. lactis and L.lactis ssp. cremoris strains as indicated in Table A were examined for secreted proteins by analysing culture supernatant fractions using SDS/PAGE [Laemmli, 1970 loc.cit.]. As shown in Fig. 1A, all of the tested strains secrete proteins, the protein which occurred predominantly having an apparent molecular weight of 50-60 kDa. The amount of the secreted protein was estimated at 2-4 $\mu g/ml/A_{600}$.

In order to investigate whether these 50-60 kDa proteins displayed a mutual relationship, antibodies against the "60 kDa protein", which was isolated from the plasmid-free L.lactis ssp. lactis strain MG1363, were generated in rabbits. Western blot analysis [Towbin et al., Proc. Natl. Acad. Sci. USA 76 (1979), 4350-4354] of the culture supernatant fractions showed a strong reaction between the 50-60 kDa proteins of all investigated strains and the isolated antibodies (Fig. 1B), which indicates that there is an immunological relationship between these proteins. It was not possible to detect any 50-60 kDa proteins in the cell extracts of the investigated strains, which indicates that these proteins are secreted efficiently into the extracellular medium and are not associated with the cell envelope, which is the case for components of the proteolytic system of lactococci [Vos et al., 1989b, loc.cit. and Kok et al., 1985, loc.cit.].

Extracellular proteins of Leuconostoc paramesenteroides, Streptococcus thermophilus, Lactobacillus casei, Lb.acidophilus and Lb.bulgaricus strains showed no reaction with the abovementioned antibodies, which indicates that the protein is very specific for lactococci sub-strains and has no immunological counterpart in other lactic acid bacteria.

### (b) Cloning and bringing to expression of the gene of the 60 kDa protein in E.coli

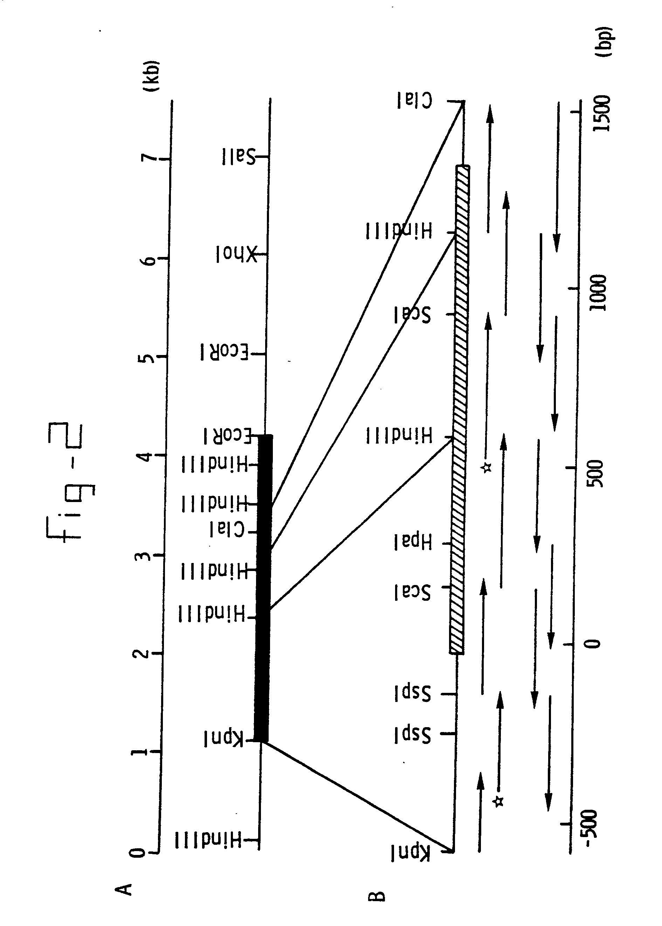

As the plasmid-free Lactococcus lactis ssp. lactis strain MG1363 is capable of producing the 60 kDa protein, chromosomal DNA of this strain was taken as starting material for cloning the gene of the 60 kDa protein. A genome library of L.lactis ssp. lactis MG1363 was constructed and screened using the abovementioned isolated antibodies. A restriction enzyme digestion map of the DNA insert of one of the phages which reacted with antibodies is given in Fig. 2.

In order to verify whether the gene for the 60 kDa protein was present in this insert, Southern blot analysis was carried out on the insert DNA. For this purpose the 10 amino-terminal amino acids of the "mature" fragment were determined using a gas phase sequenator (Applied Biosystems). The sequence was found to be Asp-Thr-Asn-Ser-Asp-Ile-Ala-Lys-Gln-Asp (D-T-N-S-D-I-A-K-Q-D). Mixed oligonucleotide probes were derived from the final six amino acids of this sequence, which had the least degenerate codon usage. Hybridisation studies of the digested phage-DNA using these probes indicated that the gene for the 60 kDa protein was located on a KpnI/EcoRI fragment of about 3 kb (Fig. 2A).

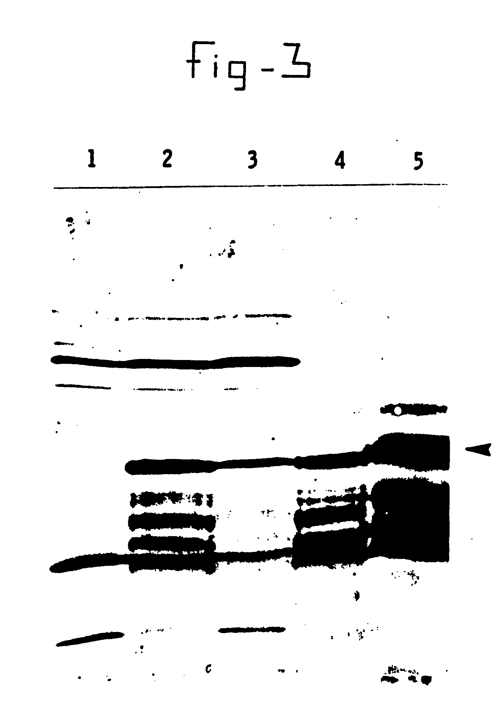

In order to demonstrate that this fragment contained the complete gene for the 60 kDa protein, it was cloned in pUC19, which gave pNZ1011. Western blot analysis of cell extracts of E.coli JM83 with pNZ1011 demonstrated the synthesis of the protein (Fig. 3, lane 2). Moreover, an appreciable amount of the 60 kDa protein could be detected in the periplasmatic fraction of the cells (lane 4). In order to investigate whether the small amount of 60 kDa protein which was still present in the stripped cells (lane 3) was due to an incomplete isolation of the periplasmatic fraction, β-lactamase determinations [Miller, 1972, loc.cit.] were carried out. The ratio of β-lactamase activity in the periplasmatic fraction compared with the stripped cells (4:1) was in complete agreement with the amounts of the 60 kDa protein which was detected in these two fractions. On these grounds it could be concluded that the 60 kDa protein is transported to the periplas-

7

matic space of E.coli cells.

It can be deduced from the above that the regulatory sequences of this Lactococcus gene and the signal peptide of the gene product are recognised in E.coli.

### (c) Nucleotide sequence of the gene for the 60 kDa protein

The nucleotide sequence of the KpnI/ClaI fragment from pNZ1011 was determined (Fig. 4) using the sequence strategy as indicated in Fig. 2B. The DNA sequence which codes for the first 10 N-terminal amino acids of the "mature" fragment was found at nucleotide position 97-113 and forms the start of an open reading frame which coded for a "mature" fragment of 434 amino acids. This protein had a predicted molecular weight of 44628 Da and, as already mentioned above, was designated "Usp45".

Three in-frame initiation codons are located upstream of the "mature" N-terminus. The most probable initiation codon is ATG at position 1 (see Fig. 4), since it precedes a precursor region which has the characteristics of a signal peptide: various basic residues followed by a hydrophobic core of 15-20 residues and a shorter uncharged region which ends with an amino acid having a short side chain [Von Heijne, 1985, loc.cit.]. Although the highest cleavage probability for the signal peptidase I is on the C-terminal side of Ala-18, according to the Von Heijne (1986) rules, the cleavage detected downstream of Ala-27 in the sequence Val-X-Ala is also commonly found in prokaryotes, in particular in Gram-positive strains [Von Heijne and Abrahmsen, FEBS Letters 244 (1989), 439-446].

The ATG start codon in position 1 is preceded by two potential ribosome binding sites [Shine and Dalgarno, Proc. Natl. Acad. Sci. USA 71 (1974), 1342-1346], namely the sequence GGAGG in position -26 to -22 and AAAG at -10 to -8. The calculated free energy of the latter is only -4.6 kcal/mol [Tinoco et al., Nature 246 (1973), 40-41]. Ribosome binding sites of genes from lactococci [De Vos. FEMS Microbiol. Rev. 46 (1987), 281-295] and other Gram-positive organisms such as Bacilli [McLauglin et al., J. Biol. Chem. 256 (1981), 11283-11291] usually have free energy values of between -10 and -15 kcal/mol. A free energy value of -14.4 kcal/mol could be calculated for the other possibility. However, the spacing to the ATG codon is 21 bp. This length does not fit in the Shine and Dalgarno hypothesis.

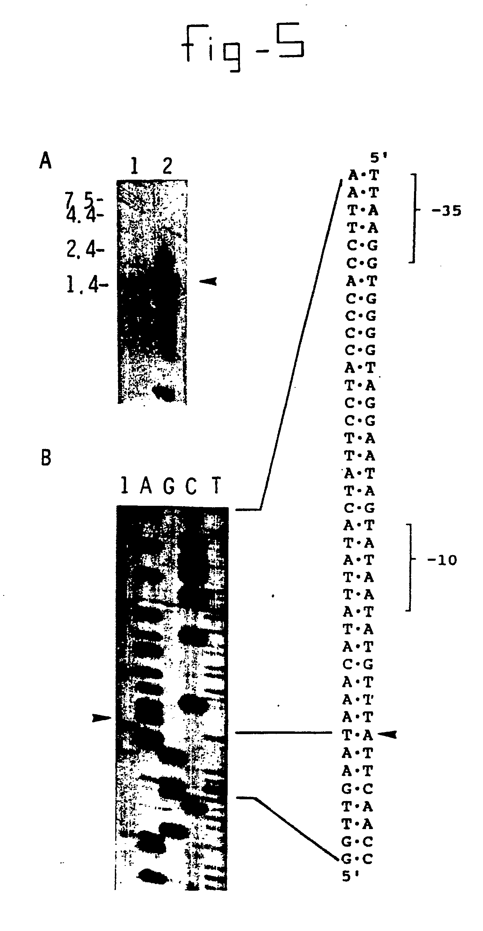

Primer extension experiments [Debarbouille and Raibaud, J. Bacteriol. 153 (1983), 1221-1227] were carried out (Fig. 5B) and showed that the transcription of the usp45 gene was initiated with an adenine residue at nucleotide position -44. A consensus -10 hexanucleotide sequence (TATAAT) was found at position -56 to -51. The most probable corresponding -35 sequence is TTAAGG (-76 to -71), although the spacing between the two is smaller (14 bp) than is usually found in promoters in E.coli [Hawley & McClure, Nucl. Acids. Res. 11 (1983). 2237-2255] and L.lactis [De Vos, FEMS Microbiol. Rev. 46 (1987), 281-295 and Van der Vossen et al., Appl. Environ. Microbiol. 53 (1987), 2452-2457].

Inspection of the DNA sequence showed the presence of several repetitive sequences: the hexanucleotide sequence GC[A/C/G/T]CAA occurs in the gene 19 times, 16 times of which are in the open reading frame, while the more degenerated sequence TC[A/T]AG[C/T] occurs 16 times. Translation of this sequence results in 12 serine doublets, 7 of which occur consecutively in one part of the Usp45, which results in an unusual amino acid sequence in the protein in question.

Downstream of the TAG stop codon of the usp45 gene, an inverted repeat was found at nucleotide position 1414 to 1452, which can form a hairpin-like structure with a free energy of -8.2 kcal/mol [Tinoco et al., Nature 246 (1973), 40-41]. The hairpin formed is flanked on either side by A or T stretches and for this reason should be able to act as rho-independent, bidirectional terminator of the transcription. Northern blot analysis revealed a messenger RNA of 1500 nucleotides (Fig. 5A), which corresponds to the deduced transcription termination.

### (d) Amino acid sequence of the Usp45 protein

The amino acid sequence of Usp45, as shown in Fig. 4, discloses a high content of serine and alanine residues, together more than 32%. In one region of the protein, i.e. the residues 264-316, serine residues form 33 of the 53 residues, including a continuous series of 16 residues.

### (e) Secretion of prochymosin by L.lactis

Plasmid pNZ1086 was introduced into L.lactis strain MG1363 by means of electroporation using the Biorad Gene Pulser TM in combination with the Gene Controller TM as described by Simons et al. Dev. in Industrial Microbiology vol. 31, 31-39.

Supernatant fractions of L.lactis cultures were analysed on 10% SDS/PAGE, blotted on nitrocellulose filters, incubated with chymosin antibodies (Chr. Hansen's Laboratorium A/S, Denmark) and stained (see Materials and Methods section c).

A protein band reacting with the chymosin antibodies and with a similar size as prochymosin could be identified in culture supernatant fractions.

## (f) Secretion of α-amylase by L.lactis

Plasmid pNZ10α1 was transformed to L.lactis cells as described above. α-Amylase producing colonies could be identified on agar plates containing 0.5% starch and subsequently staining with a KI/I$_2$ solution. A large halo surrounding the colonies was found. Furthermore, culture supernatant fractions were characterised by Western blot analysis, using antibodies raised in rabbits against α-amylase of Bacillus stearothermophilus (Cerestar, Belgium). A protein band reacting with these antibodies and with a similar size as α-amylase of Bacillus stearothermophilus could be identified in culture supernatant fractions.

A seven fold increase in α-amylase activity compared to L.lactis strain MG1363, harbouring pNZ10α1 was found in supernatant fractions of L.lactis cells containing plasmid pNZ10αA.

## LEGENDS

**Fig. 1**: Gel analysis of extracellular proteins from L.lactis strains.

Supernatants of logarithmically growing cultures were dialysed against water and concentrated by lyophilisation. Samples were analysed using 10% SDS/PAGE [Laemmli, 1970, loc.cit.] and Western blotting.

Panel A shows a Coomassie brilliant blue staining of 1.0 ml supernatant. Lanes: (1) MG1363, (2) IL1403, (3) R1, (4) NZ22186, (5) NCD0176, (6) SK1128, (7) NCD01200, (8) WG2. The position of the 50-60 kDa proteins is indicated. The lanes are flanked on the left-hand side by molecular weight markers, expressed in kDa.

Panel B is a Western blot of a gel which is loaded with 0.2 ml of the same samples, obtained after incubation with an antibody against the 60 kDa protein of MG1363. The location of the 50-60 kDa protein is indicated.

**Fig. 2**: restriction map and sequencing strategy for the gene which codes for the 60 kDa protein of MG1363.

(a) Restriction map of the inserted DNA of a positive phage clone. Hybridisation with the mixed oligonucleotide series 5'-GA[C/T]-AT[A/C/T]-GCN-AA[A/G]-CA[A/G]-3' as probe is indicated by a black bar.

(b) Restriction map and sequencing strategy for the KpnI/ClaI fragment of pNZ1011. The map shows the coding region of the gene (hatched bar). The arrows show the length and direction of the individual sequenced stretches. M13 primers and synthetic oligo-primers (indicated by *) were used. The sequence was determined using the dideoxychain termination method [Sanger, Proc. Natl. Acad. Sci. USA 74 - (1977), 5463-5467] carried out in accordance with the sequenase protocol [Tabor & Richardson, Proc. Natl. Acad. Sci. USA 84 (1987), 4767-4771].

**Fig. 3**: Analysis of the proteins, encoded by E.coli JM83 cells with pNZ1011.

The cells were fractionated with the aid of an osmotic shock [Neu & Heppel, J. Biol. Chem. 240 (1965), 3685-3692], which resulted in a periplasmatic fraction and a stripped cell fraction. The Western blot was incubated with the antibody against the 60 kDa protein from MG1363. Lanes: (1) JM83 with pUC19, non-fractionated cells, (2), (3) and (4) JM83 with pNZ1011. (2) non-fractionated cells, (3) stripped cells, (4) periplasmatic fraction. (5) MG1363, culture supernatant. The position of the 60 kDa protein is indicated.

**Fig. 4**: Nucleotide sequence of the usp45 gene and the deduced amino acid sequence. The nucleotide sequence of the region upstream the usp45 gene starting at the KpnI site of Fig. 2 is also included.

**Fig. 5**: RNA analysis and Northern blotting

A: The total RNA was analysed on a glyoxal-agarose gel [Thomas, 1980] and blotted on a Genescreen filter (NEN/Dupont). The blot was then investigated using an M13-generated probe, which is complimentary to the usp45 gene.

Lanes: (1) 5 μg total RNA, (2) 20 μg total RNA. The 1.5 kb transcript is indicated. The positions of the RNA markers (BRL) are indicated in kb.

B: Determination of the transcription initiation of the usp45 gene.

cDNA was generated by reverse transcriptase elongation using the oligoprimer 5'CAGCAGAAAGTATCACTG 3' (nucleotide position 55 to 37). The sequence order, obtained using the same primer, was used as marker. The corresponding DNA sequence is shown and the position of the transcription initiation site and the -10 and -35 boxes are shown.

**Fig. 6**: Schematic representation of the construction of plasmid pNZ1086.

Cm, Ap, chloramphenicol and ampicillin resistance genes, respectively; ori, origin of replication. The direction of transcription of the Cm, Ap, usp45 and bovine prochymosin gene is indicated with an arrow. The size of the plasmid and DNA fragments is indicated. Only relevant restriction sites are shown. The nucleotide sequence at the junctions was verified by DNA sequence analysis.

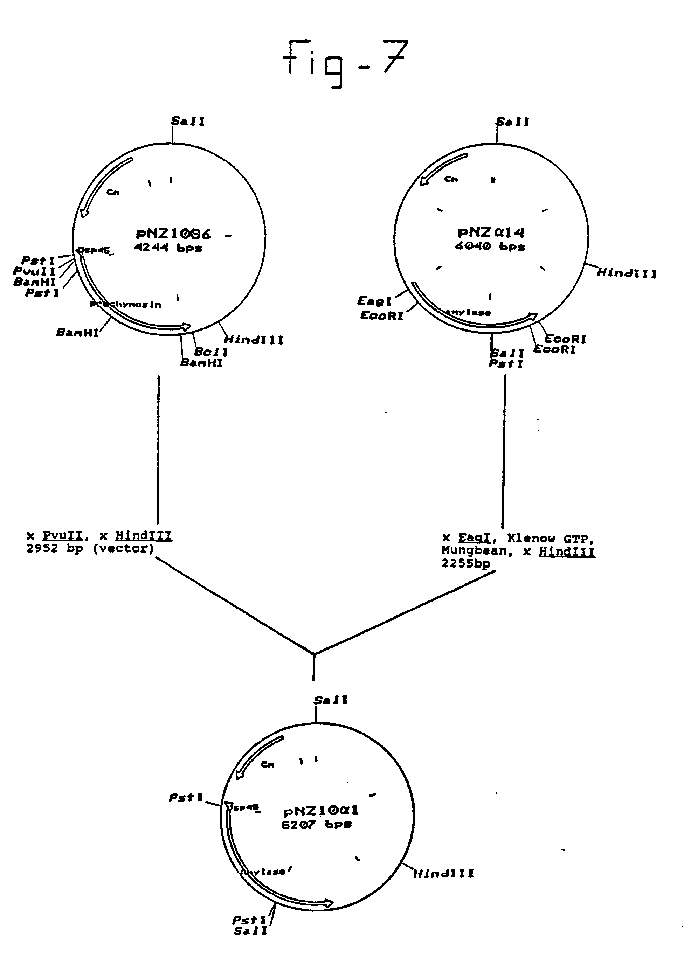

**Fig. 7**: Schematic representation of the construction of plasmid pNZ10α1.

Cm, chloramphenicol resistance genes; ori, origin of replication. The direction of transcription of the Cm, usp45 and α-amylase gene of Bacillus stearothermophilus is indicated with an arrow. The size of the plasmid and DNA fragments is indicated. Only relevant restriction sites are shown. The nucleotide sequence at the junctions was verified by DNA sequence analysis.

## Claims

1. DNA fragment, comprising at least part of the sequence which is located upstream of the sequence coding for the "mature" fragment of the extracellular protein having an apparent molecular weight of 60 kDa from Lactococcus lactis strains.

2. DNA fragment according to Claim 1, characterised in that it comprises at least part of the sequence which is located upstream of the sequence coding for the "mature" fragment of the extracellular 60 kDa protein from the Lactococcus lactis ssp. lactis strain MG1363.

3. DNA fragment according to Claim 1 or 2, characterised in that it comprises at least part of the sequence located upstream of the sequence coding for the "mature" fragment of the extracellular 60 kDa protein, which "mature" fragment starts with D (28) in accordance with Fig. 4.

4. DNA fragment according to Claim 3, characterised in that the fragment comprises at least the signal sequence which codes for M (1) to A (27) inclusive, as shown in Fig. 4.

5. DNA fragment according to one or more of Claims 1-4, characterised in that the fragment also comprises the sequence coding for the "mature" fragment of the extracellular protein having an apparent molecular weight of 60 kDa.

6. DNA fragment according to Claim 5, characterised in that the fragment comprises the sequence shown in Fig. 4.

7. DNA fragment according to one or more of Claims 1-6, characterised in that the fragment also comprises an extra sequence which codes for a homologous or a heterologous protein.

8. DNA fragment according to Claim 7, characterised in that the homologous protein is a component of the proteolytic system of Lactococcus lactis such as a proteinase or peptidase.

9. DNA fragment according to Claim 7, characterised in that the heterologous protein is pseudo-chymosin, prochymosin, chymosin or α-amylase.

10. Plasmid, which contains at least a DNA fragment according to one or more of Claims 1-9.

11. Host, preferably a lactococcus strain, which comprises in the genome a DNA fragment according to one or more of Claims 5-9 or a plasmid according to Claim 10.

12. Protein, obtained by culturing a host according to Claim 11 and isolating the extracellular protein from the culture medium.

# fig -1

EP 0 455 280 A1

# Fig-2

A

0    1    2    3    4    5    6    7    (kb)

HindIII   KpnI   HindIII   HindIII   ClaI   HindIII   HindIII   EcoRI   EcoRI   XhoI   SalI

B

KpnI   SspI   SspI   ScaI   HpaI   HindIII   ScaI   HindIII   ClaI

☆

☆

-500    0    500    1000    1500    (bp)

# Fig-3

# Fig-4 (1)

EP 0 455 280 A1

```
                                                              KpnI
                                                              GGTACCAGTGAGTCATTTATTTTTGATTTTTTCTAATGTATAGTG

TCACTCAGCACAGGCCAAGCTATATCATTATCTTAGCGGCGATTTTGGGTGCAATCTATGATAGCTATTATTTAGGTATTTACGGAATTGCGACCTTATTGTTCCCACTTATTGCTCTTT

TTGTATATAATATACAAATAACTATATTTACTAATCGCTGGACAAGGCTTTTTACAACAATTATTATTGTGACCGCTTTTGAAGTTTTTAGTGCAATCATTATGACAGCTTTTGGATTTG
                                         HaeII                    SspI
CCCAACTTCAGTTTATCAAATTTGTTGTTTACCAGTTAGCGCCTACACTTTTGCTCAATATTATCTTAGCTGTAGCCTTACAATTCCCTTTAGAAATCTTTTACAGATTAAAGAAAAGTC
             SspI
ATGTAAGATACAATTAGAAAGTGTTTTGTAATCATAAAGAAATATTAAGGTGGGGTAGGAATAGTATAATATGTTTATTCAACCGAACTTAATGGGAGGAAAAATTAAAAAAGAACAGTT

1
ATGAAAAAAAAGATTATCTCAGCTATTTTAATGTCTACAGTGATACTTTCTGCTGCAGCCCCGTTGTCAGGTGTTTACGCTGACACAAACTCAGATATTGCTAAACAAGATGCGACAATT

  1 M K K K I I S A I L M S T V I L S A A A P L S G V Y A D T N S D I A K Q D A T I

121
TCAAGCGCGCAATCTGCTAAAGCACAAGCACAAGCACAAGTTGATAGCTTGCAATCAAAAGTTGACAGCTTACAACAAAAGCAAACAAGTACTAAAGCACAAATCGCTAAAATCGAAAGC

 41 S S A Q S A K A Q A Q A Q V D S L Q S K V D S L Q Q K Q T S T K A Q I A K I E S

241
GAAGCTAAAGCACTTAATGCTCAAATTGCTACTTTGAACGAAAGTATCAAAGAACGTACAAAGACATTGGAAGCTCAAGCACGTAGTGCTCAAGTTAACAGCTCAGCAACAAATTATATG

 81 E A K A L N A Q I A T L N E S I K E R T K T L E A Q A R S A Q V N S S A T N Y M

361
GATGCTGTTGTTAATTCAAAATCTTTGACAGATGTTATTCAAAAAGTAACAGCTATTGCTACTGTTTCTAGTGCCAACAAACAAATGTTGGAACAACAAGAAAAAGAGCAAAAAGAGCTT

121 D A V V N S K S L T D V I Q K V T A I A T V S S A N K Q I L E Q Q E K E Q K E L

481
AGCCAAAAAGTCAGAAAACTGTTAAAAAAGAACTACAACCAGTTCGTTTCTCTTTCACAAAGTTTGGATTCTCAAGCTCAAGAATTGACTTCACAACAAGCTGAACTCAAAGTTGCGACTTTG

161 S Q K S E T V K K N Y M Q F V S L S Q S L D S Q A Q E L T S Q Q A E L K V A T L
```

## Fig-4 (2)

```
601
AACTATCAAGCAACAATTGCAACTGCGCAAGATAAAAAACAAGCTTTATTAGATGAAAAAGCAGCTGCAGAAAAAGCAGCTCAAGAAGCAGCTAAAAAACAAGCGGCTTATGAAGCTCAA

201  N  Y  Q  A  T  I  A  T  A  Q  D  K  K  Q  A  L  L  D  E  K  A  A  A  E  K  A  A  Q  E  A  A  K  K  Q  A  A  Y  E  A  Q

721
CAAAAAGAAGCAGCACAAGCACAAGCAGCTTCAACAGCAGCAACTGCTAAAGCTGTAGAAGCAGCAACTTCATCAGCTTCTGCTTCATCTAGTCAAGCTCCACAAGTAAGTACAAGCACT

241  Q  K  E  A  A  Q  A  Q  A  A  S  T  A  A  T  A  K  A  V  E  A  A  T  S  S  A  S  A  S  S  Q  A  P  Q  V  S  T  S  T

841
GATAATACAACATCAAATGCTAGTGCCTCAAACAGTTCTAATAGTTCATCAAACTCAAGTTCAAGTTCTAGCAGTTCATCAAGCTCAAGCTCAAGCTCAAGTAATTCTAATGCTGGTGGG

281  D  N  T  T  S  N  A  S  A  S  N  S  S  N  S  S  S  N  S  S  S  S  S  S  S  S  S  S  S  S  S  S  S  N  S  N  A  G  G

961
AATACAAATTCAGGCACTAGTACTGGAAATACTGGAGGAACAACTACTGGTGGTAGCGGTATAAATAGTTCACCAATTGGAAATCCTTATGCTGGTGGTGGATGTACTGACTATGTATGG

321  N  T  N  S  G  T  S  T  G  N  T  G  G  T  T  T  G  G  S  G  I  N  S  S  P  I  G  N  P  Y  A  G  G  C  T  D  Y  V  W

1081
CAATACTTTGCTGCACAAGGAATTTATATCAGAAATATCATGCCTGGTAATGGTGGACAATGGGCTTCTAATGGACCTGCCCAAGGCGTGCTCCATGTTGTAGGAGCTGCTCCTGGTGTT

361  Q  Y  F  A  A  Q  G  I  Y  I  R  N  I  M  P  G  N  G  G  Q  W  A  S  N  G  P  A  Q  G  V  L  H  V  V  G  A  A  P  G  V

1201
ATCGCATCAAGCTTCTCAGCTGATTTTGTTGGATATGCAAACTCACCTTACGGTCACGTAGCTATTGTAAAATCAGTTAATTCAGATGGTACAATTACTATCAAAGAAGGCGGATATGGT

401  I  A  S  S  F  S  A  D  F  V  G  Y  A  N  S  P  Y  G  H  V  A  I  V  K  S  V  N  S  D  G  T  I  T  I  K  E  G  G  Y  G

1321
ACAACTTGGTGGGGACATGAACGTACTGTAAGTGCGTCTGGTGTTACTTTCTTGATGCCAAACTAGAAAAAAGTCTTAATAAATAAAAAATAGTGGTTTGATAGTGGGGAATAATTTTCC

441  T  T  W  W  G  H  E  R  T  V  S  A  S  G  V  T  F  L  M  P  N                                    ------------------>              <-

1441
TTCTGTCAAATCATTTTTTATTATTGTGGTATAATAATAAGGAAAAATGATA
--------------
```

EP 0 455 280 A1

# Fig-5

# Fig-6

# fig-7

x PvuII, x HindIII
2952 bp (vector)

x EagI, Klenow GTP,
Mungbean, x HindIII
2255bp

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 000 558 (UNIVERSITY OF MARY-LAND)(25-01-1990) <br> * Claims; discussion * <br> – – – | 1 | C 12 N 15/31 <br> C 12 N 15/62 <br> C 12 N 15/52 <br> C 12 N 1/21 // <br> (C 12 N 1/21 <br> C 12 R 1:46 ) |
| A | CHEMICAL ABSTRACTS, vol. 109, 1988, pages 152, abstract no. 1522c, Cclumbus, Ohio, US; J. KOK et al.: "Nucleotide sequence of the cell wall proteinase gene of Streptococcus cremoris Wg2", <br> & APPL. ENVIRON. MICROBIOL. 1988, 54 (1), 231-8 <br> – – – | 1 | |
| A | NETH. MILK DAIRY J., vol. 43, no. 3, 1989, pages 327-345; H. LAAN et al.: "Enzymes involved in the degradation and utilization of casein in Lactococcus lactis" <br> * Whole article * <br> – – – – – | 1 | |
| | | | **TECHNICAL FIELDS SEARCHED (Int. Cl.5)** <br><br> C 07 K <br> C 12 N |

The present search report has been drawn up for all claims

| Place of search | Date of completion of search | Examiner |
|---|---|---|
| The Hague | 10 July 91 | DELANGHE L.L.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document